**Europäisches Patentamt**

**European Patent Office**

**Office européen des brevets**

(11) Numéro de publication : **0 561 968 B1**

(12)                          **FASCICULE DE BREVET EUROPEEN**

(45) Date de publication du fascicule du brevet :
23.11.94 Bulletin 94/47

(51) Int. Cl.⁵ : **G01N 33/24,** G01N 3/48

(21) Numéro de dépôt : **92901776.2**

(22) Date de dépôt : **10.12.91**

(86) Numéro de dépôt international :
**PCT/FR91/00997**

(87) Numéro de publication internationale :
**WO 92/10753 25.06.92 Gazette 92/14**

(54) **PENETROMETRE DYNAMIQUE PYROTECHNIQUE.**

(30) Priorité : **12.12.90 FR 9015512**

(43) Date de publication de la demande :
**29.09.93 Bulletin 93/39**

(45) Mention de la délivrance du brevet :
**23.11.94 Bulletin 94/47**

(84) Etats contractants désignés :
**AT BE CH DE DK ES FR GB GR IT LI LU NL SE**

(56) Documents cités :
**EP-A- 389 375
FR-A- 2 290 660
FR-A- 2 584 186
US-A- 3 894 426
US-A- 4 492 111**

(73) Titulaire : **Castagner, Bernard
4 Chemin des Bourgognes
F-77450 Coupvray (FR)**

(72) Inventeur : **Castagner, Bernard
4 Chemin des Bourgognes
F-77450 Coupvray (FR)**

(74) Mandataire : **Michelet, Alain et al
Cabinet Harlé et Phelip
21 rue de la Rochefoucauld
F-75009 Paris (FR)**

## Description

La présente invention est relative à un système de reconnaissance des sols et de mesure de leurs caractéristiques. Les deux principaux systèmes de reconnaissance des sols existants sont la reconnaissance par pressiométrie et la reconnaissance par pénétrométrie en particulier la pénétrométrie dynamique. Le principe de la pénétrométrie dynamique est de déterminer l'énergie cinétique nécessaire pour enfoncer une sonde d'une certaine hauteur, l'énergie cinétique étant en général créée par la chute calibrée d'une masse donnée.

Les sondages effectués avec ce système prennent beaucoup de temps et en notre ère de coût de main d'oeuvre élevée, ils sont onéreux.

Par ailleurs, la plupart de ces systèmes n'intègrent pas un relevé automatique écrit des caractéristiques des sols, ce qui conférerait une plus grande crédibilité aux reconnaissances effectuées.

Le système de pénétrométrie dynamique pyrotechnique objet de l'innovation permet de répondre, en particulier à ces demandes majeures : rapidité d'exécution, diminution des coûts et crédibilité de la mesure.

Le système objet du présent brevet détermine les caractéristiques du sol par l'enfoncement détecté et mesuré en continu, d'une tige sonde étalon dans le sol. Cet enfoncement est réalisé par l'utilisation de la pression créée par un générateur de gaz pyrotechnique.

L'originalité de la conception du système, objet du présent brevet, ressortira de sa description, de la justification des techniques utilisées et de son diagramme de fonctionnement.

Les particularités importantes du système proposé par l'invention sont les suivantes :

- On utilise la pression créée par un générateur de gaz pyrotechnique dans un tube d'éjection pour enfoncer une tige sonde étalon dans le sol.
- On détecte en continu le déplacement de la tige dans le tube d'éjection, en particulier par le passage d'un aimant lié à la tige dans des solénoïdes placés le long du tube d'éjection.
- On calcule en continu, à l'aide d'un circuit électronique pré-programmé la résultante accélérométrique de ce déplacement qui est la résultante de l'accélération créée par la pression du gaz sur la tige et de l'accélération créée par la résistance du sol sur cette même tige.
- On utilise dans le cas d'un système léger et portatif, l'équilibrage des quantités de mouvements par une masse reculante verticale dont une partie sert préférentiellement de chambre de détente des gaz et de guide de la tige étalon.

La description qui va suivre est purement illustrative et non limitative, cela doit être lu en regard des dessins annexés :

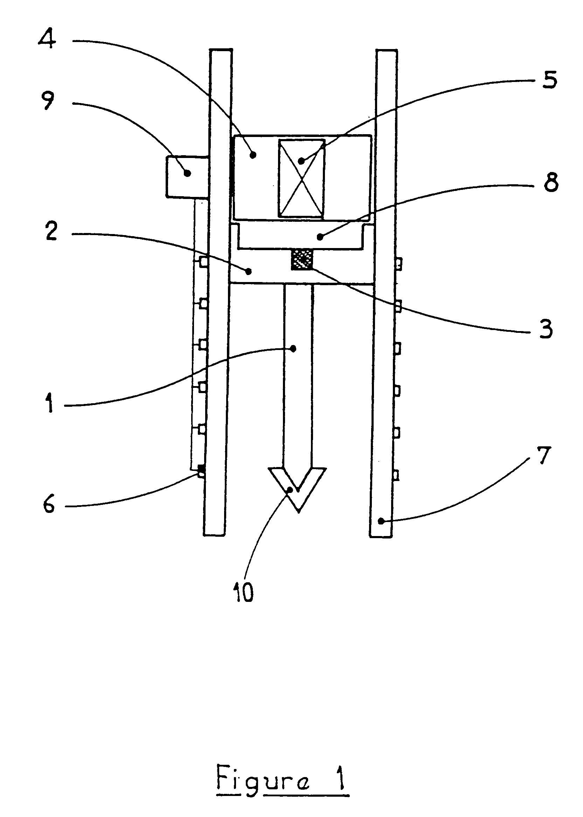

- La figure 1 : représente l'expression la plus simple du système objet de l'invention.
- La figure 2 : représente un système plus spécialement adapté à des sondages de faible profondeur.
- Les figures 3 et 4 : représentent un système plus spécialement adapté à des sondages de plus grande profondeur. Le système de base est schématisé par la figure 1.

La tige sonde étalon (1) est fixée sur un piston (2) dans lequel on place un aimant (3).

La masse reculante (4) équilibre la quantité de mouvement qui sera transmise à la tige étalon (1) et à son piston (2).

Elle est équipée d'un générateur de gaz (5). L'absorption de l'énergie de recul est assurée par la gravité.

Sans sortir du cadre de l'invention, on peut utiliser un autre moyen pour équilibrer les efforts de recul, tels que absorbeurs d'énergie, masse du porteur ou du système.

L'ensemble tige, piston et masse reculante, générateur est placé dans un tube de recul (7) fabriqué en matériaux amagnétiques. Le tube de recul (7) est équipé de solénoïdes (6) préférentiellement placés concentriques au tube de lancement.

Ces solénoïdes (6) sont reliés au boîtier électronique et informatique de traitement des informations (9).

Après allumage, le générateur de gaz débite dans la chambre de détente (8) située entre le piston (2) et sa masse reculante (4), la pression ainsi générée, met en vitesse la tige étalon (1). Au fur et à mesure de l'éjection de la tige, le passage de l'aimant (3) dans les différents solénoïdes (6) génère des signaux électriques dont on calcule les intervalles d'apparition.

Ceci permet de calculer la vitesse puis l'accélération de la tige étalon.

La loi de pression P(t) délivrée par un générateur de gaz donné dans la chambre de détente est connue : soit précisément du fait d'une mesure, soit avec une bonne approximation (2 à 3%) du fait de la reproductibilité des performances du générateur de gaz utilisé.

La force de décélération due à la résistance du sol à l'enfoncement de la tige étalon est donc connue :

$$R(t) = S.P.(t) = m\,G(t)$$

Les calculs sont effectués soit avec un circuit logique adapté, soit avec un calculateur existant pré-programmé.

Sans sortir du cadre de l'invention, on pourra utiliser d'autres systèmes de détection du passage de ce type, tels que des contacteurs électromécaniques ou des cellules photo-électriques.

De même, sans sortir du cadre de l'invention, on peut replacer les détecteurs de passage et une partie du circuit de traitement des informations par un accéléromètre.

Selon le type de mesure à effectuer, la tige étalon peut être ou non équipée d'une pointe débordante (10).

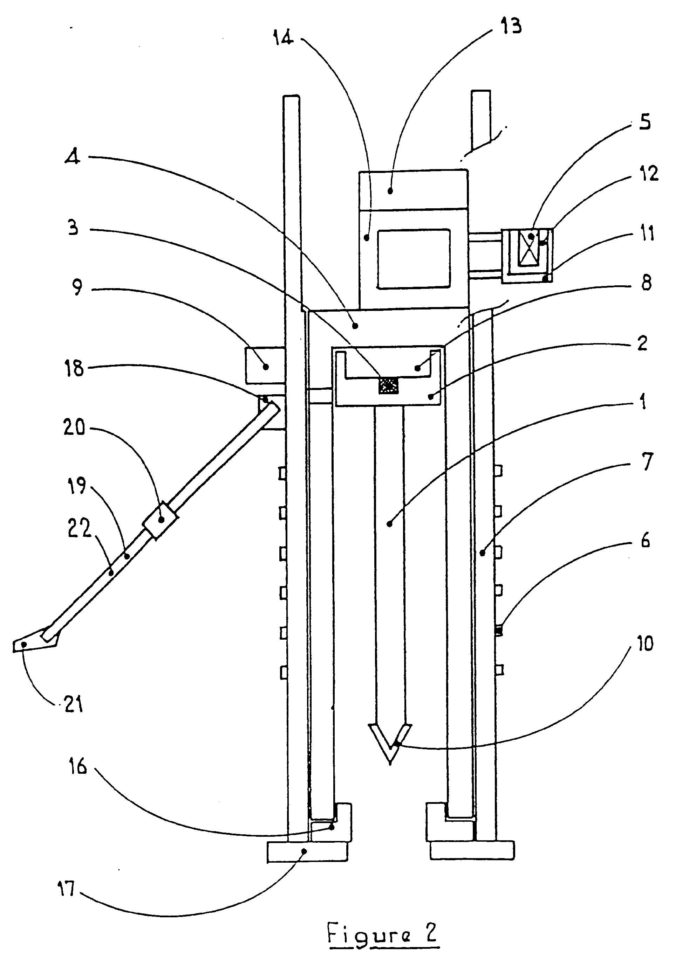

Le système objet de la présente invention dans sa version portable et opérationnelle pour des sondages de faibles profondeurs est schématisé par la figure 2.

La tige sonde étalon (1) est fixée par un piston (2) dans lequel on place un aimant.

La masse reculante (4) équilibre la quantité de mouvements qui sera transmise à la tige étalon et à son piston. Elle est préférentiellement constituée d'un tube en matériaux amagnétiques par exemple tube cylindrique fermé à son extrémité supérieure, la masse reculante sert ainsi de tube d'éjection.

Avant l'enfoncement, la tige étalon équipée du piston est maintenue dans la masse reculante préférentiellement à l'aide d'un clip déformable (15) mais sans sortir de l'invention, on peut utiliser un autre système de fixation tel que ressort à bille, ressort à lame...

La masse reculante (4) coulisse dans un tube de recul (7) en matériaux amagnétiques préférentiellement en composite fibre de verre à matrice thermodurcissable ou thermoplastique.

Au niveau de son contact avec le sol, le tube de guidage est préférentiellement doté d'un talon d'appui (17) équipé d'un amortisseur (16) de retombée de la masse reculante.

Les solénoïdes (6) de détection de passage de l'aimant sont situés préférentiellement autour du tube de guidage et relié au boîtier électronique de traitement (9).

Le système peut être autonome et être simplement équipé d'un trépied (22) repliable et réglable.

Ce trépied peut préférentiellement être constitué d'une bague d'assemblage (18) des trois pieds (19) de longueur réglable grâce par exemple à un coulisseau verrouillable (20) et prenant appui sur le sol à l'aide de patin (22) qui peuvent être troués pour éventuellement fixer le système au sol.

La masse reculante est préférentiellement surmontée du support de la culasse (17) du générateur de gaz et de son système de sécurité d'initiation (13).

L'initiation du générateur de gaz est préférentiellement à percussion mais sans sortir du cadre de l'invention, on pourra utiliser une initiation électrique ou par faisceau laser.

Le corps du générateur de gaz est préférentiellement en matériaux thermoplastique, thermodurcissable, élastomère, carton ou composite et sa structure est préférentiellement conçue pour s'ouvrir à basse pression hors de son logement d'utilisation et ce pour des raisons de sécurité en cas d'éventuel allumage accidentel ou d'incendie.

Pour faciliter le chargement et assurer le sécurité, le générateur de gaz (5) est préférentiellement placé dans un logement (12) situé dans une culasse mobile (11).

L'introduction de cette culasse mobile (11) dans son support (14) permet de placer le générateur de gaz en position fonctionnement face au canal d'entrée à la chambre de détente (8) et d'armer le système de sécurité d'initiation (13).

Sans sortir du cadre de l'invention, on peut adapter d'autre construction de la fonction "piston-éjection".

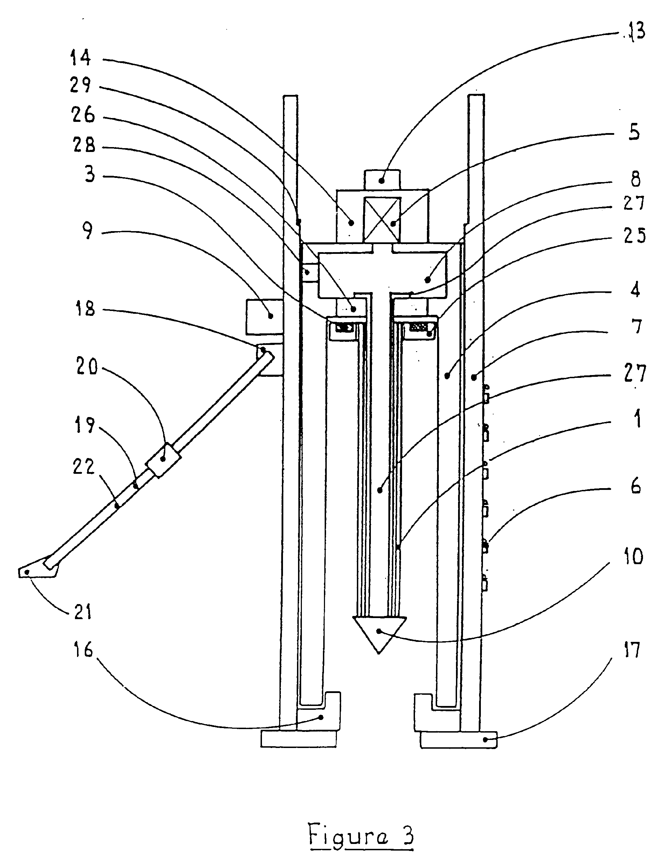

La figure 3 présente une variante spécialement adaptée au sondage profond effectué à l'aide d'une seule tige étalon ou de plusieurs tiges assemblées au fur et à mesure de l'exécution du sondage.

La fonction poussée éjection et la fonction guidage de la tige étalon ne sont pas assurées par un piston mais d'une part par l'extrémité fermée de la tige étalon creuse (1) portant la pointe débordante (10) ou non débordante, et d'autre part par un tube axial le long duquel coulisse la tige étalon.

La tige sonde étalon (1) est équipée d'un talon (25) qui permet de limiter son enfoncement dans le sol, en cas excès d'énergie par rapport à l'énergie absorbée par l'enfoncement dans le sol.

Les aimants (3) sont situés dans ce talon (25). Dans ce cas, les solénoïdes ne sont pas concentriques au tube mais situés le long du tube et les aimants placés dans un plan perpendiculaire au mouvement de la tige.

La tige sonde étalon coulisse autour d'un tube guide (27) qui est lié, préférentiellement à l'aide d'un mandrin (26), à la masse reculante (4), celle-ci fait aussi tube de guidage de recul, et est équipée de la chambre de détente (8) et de la culasse du générateur de gaz (5). Sans sortir du cadre de l'invention, la tige sonde (1) peut être intérieure au tube guide (27).

Le tube de recul (7) comporte un alésage (29) dans sa partie supérieure.

La chambre de détente comporte un ou plusieurs orifices (28) équipés ou non de clapet ou de membrane de cisaillement.

En effet, en cas de blocage de la tige sonde étalon dans le sol, il faut évacuer les gaz de la chambre de détente pour limiter la remontée de la masse reculante.

On effectuera cette évacuation préférentiellement à l'aide de cet alésage (29) et de ces orifices (28). Ils sont placés de telle façon qu'ils ne dégradent que faiblement les performances du générateur de gaz.

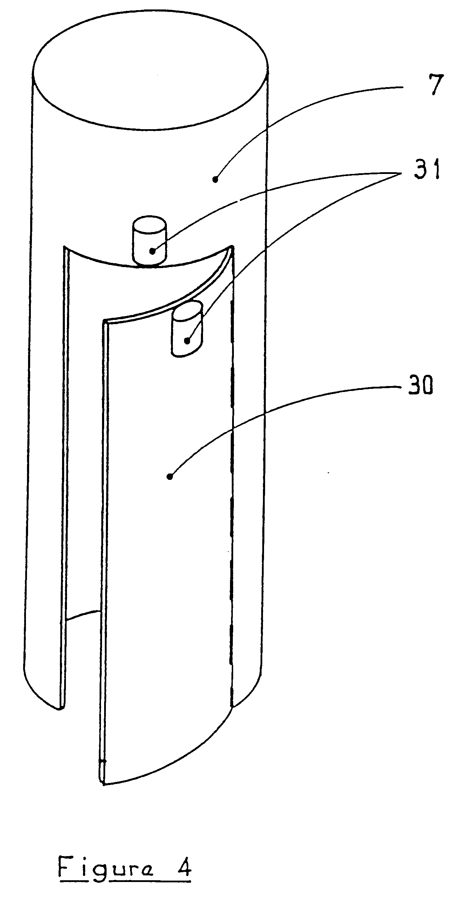

Le tube de recul (7) et la partie tubulaire de la masse reculante présente une ouverture (30) (figure 4) pour permettre le chargement de la tige étalon (1) équipée de son tube de guidage (27). Cette ouverture est obturée lors de son fonctionnement soit par un portillon (30) soit par rotation de la masse reculante dans le tube de guidage.

Dans le cas de la présence d'un portillon, le chargement de la tige sonde d'un système de verrouillage (31) qui, pour des raisons de sécurité est lié préférentiellement au dispositif de sécurité de mise à feu (15).

En cas d'enfoncement partiel ou difficile de la tige étalon, on peut aisément, soit la retirer, soit compléter son enfoncement en utilisant un second générateur de gaz.

Pour retirer la tige sonde étalon (1), il suffit d'ouvrir le mandrin (26) ; le tube guide (27) tombe au fond de la tige étalon, on peut alors dégager le système puis extraire la tige sonde, éventuellement, à l'aide d'un extracteur s'appuyant sur le talon (25).

On peut aussi ajouter une seconde tige sonde allonge pour approfondir le sondage en la fixant à la place du talon après le démontage du tube guide.

## Revendications

1. Dispositif de reconnaissance et de caractérisation d'un sol comprenant :
   - un tube de recul (7),
   - une tige sonde (1) mobile par rapport au tube de recul (7),
   - un générateur pyrotechnique,
   - des moyens de détection (6) composés de plusieurs éléments,
     . la tige sonde (1) étant destinée à être enfoncée dans le sol par l'effet du générateur pyrotechnique (5),
     . les moyens de détection (6) étant fixés sur le tube de recul (7) et chacun de leurs éléments détectant le passage de la tige sonde (i) à son niveau, de manière à fournir une mesure du mouvement de la tige sonde au cours de son enfoncement.

2. Dispositif selon la revendication 1, caractérisé en ce que le mouvement de la tige sonde (1) est détecté par le passage d'un aimant (3) lié à cette tige, dans les solénoïdes (6) placés le long du tube extérieur (7).

3. Dispositif selon la revendication 1, caractérisé en ce que le mouvement de la tige sonde (1) est détecté par le passage de celle-ci devant des cellules photo-électriques ou des contacteurs électromécaniques.

4. Dispositif selon une ou plusieurs des revendications ci-dessus, caractérisé en ce que les coordonnées de mouvements de la tige sont introduites dans un calculateur (9), spécifique ou non, pré-programmé et initialisé des paramètres du dispositif, pour obtenir directement des caractéristiques du sol et les rendre disponibles pour affichage ou impression.

5. Dispositif selon une ou plusieurs des revendications ci-dessus, caractérisé en ce que l'équilibre de la quantité de mouvements transmise à la tige sonde est assuré par une masse reculante (4) située dans le tube de recul (7), et dont l'énergie cinétique est absorbée par l'utilisation de la force de la gravité.

6. Dispositif selon une ou plusieurs des revendications ci-dessus, caractérisé en ce que la masse reculante sert de tube de guidage à la tige sonde et de chambre de détente des gaz.

7. Dispositif selon une ou plusieurs des revendications ci-dessus, caractérisé en ce que la masse reculante comporte le logement du générateur de gaz pyrotechnique (5).

8. Dispositif selon une ou plusieurs des revendications ci-dessus, caractérisé en ce que la masse reculante présente, au niveau de la chambre de détente (8), un ou plusieurs évents de sécurité (28), et le tube de recul présente un alésage (29) pour faciliter la sortie des gaz en cas de blocage prématuré de la tige sonde dans le sol lors de son enfoncement.

9. Dispositif selon une ou plusieurs des revendications ci-dessus, caractérisé par l'utilisation d'une tige sonde creuse (1) coulissante le long d'un tube guide amovible (27) bloqué dans la masse reculante (4) par un mandrin (26) ou un système de fixation rapide.

10. Dispositif selon une ou plusieurs des revendications ci-dessus, caractérisé en ce que la tige sonde peut être en plusieurs tronçons assemblés par un emboîtement-verrouillage au fur et à mesure de l'opération de sondage.

11. Dispositif selon une ou plusieurs des revendications ci-dessus, caractérisé en ce que le corps du générateur est en matériaux thermoplastique ou composite et s'ouvre à basse pression hors de son logement d'utilisation.

## Patentansprüche

1. Vorrichtung zur Charakterisierung und Erkennung eines Bodens mit :
   - einem Rückstossrohr (7),
   - einer Sondenstange (1), die sich im Verhältnis zum Rückstossrohr (7) bewegt,
   - einem pyrotechnischen Generator,

- Detektormitteln (6), die aus mehreren Teilen bestehen,
    . wobei die Sondenstange (1) dazu bestimmt ist, durch den pyrotechnischen Generator (5) in den Boden eingetrieben zu werden, und
    . wobei die Detektormittel (6) auf dem Rückstossrohr (7) befestigt sind und jedes ihrer Teile den Durchgang der Sondenstange (1) durch sein Niveau erfasst, so dass ein Mass für die Bewegung der Sondenstange während des Eindringens bereitgestellt wird .

2. Vorrichtung nach Anspruch 1, dadurch gekennzeichnet, dass die Bewegung der Sondenstange (1) durch den Durchgang eines Magneten, der mit dieser Stange verbunden ist, durch Solenoide (6), die längs des äusseren Rohres (7) angeordnet sind, erfasst wird.

3. Vorrichtung nach Anspruch 1, dadurch gekennzeichnet, dass die Bewegung der Sondenstange (1) durch deren Vorbeibewegen an Photozellen oder an elektromechanischen Schaltkontakten erfasst wird.

4. Vorrichtung nach einem der vorangehenden Ansprüche, dadurch gekennzeichnet, dass die Koordinaten der Stangenbewegung in einen speziellen oder nicht speziellen Rechner (9), der vorprogrammiert und mit den Vorrichtungsparametern initialisiert ist, eingegeben werden, um unmittelbar die Eigenschaften des Bodens zu erhalten und sie zur Anzeige oder zum Drucken verfügbar zu halten.

5. Vorrichtung nach einem der vorangehenden Ansprüche, dadurch gekennzeichnet, dass das Gleichgewicht zur Menge der auf die Sondenstange übertragenen Bewegungen durch eine Rückstossmasse (4) sichergestellt ist, wobei die genannte Rückstossmasse (7) im Rückstossrohr (7) angeordnet ist und deren kinetische Energie durch Ausnutzung der Schwerkraft absorbiert wird.

6. Vorrichtung nach einem der vorangehenden Ansprüche, dadurch gekennzeichnet, dass die Rückstossmasse als Führungsrohr für die Sondenstange und als Gasausdehnungskammer dient.

7. Vorrichtung nach einem der vorangehenden Ansprüche, dadurch gekennzeichnet, dass die Rückstossmasse das Gehäuse des pyrotechnischen Gasgenerators (5) aufweist.

8. Vorrichtung nach einem der vorangehenden Ansprüche, dadurch gekennzeichnet, dass die Rückstossmasse im Bereich der Ausdehnungskammer (8) eine oder mehrere Sicherheitsöffnungen (28) aufweist und dass das Rückstossrohr eine Bohrung (29) aufweist, um ein Entweichen von Gas im Falle eines vorzeitigen Blockierens der Sondenstange im Boden während ihres Eindringens zu erleichtern.

9. Vorrichtung nach einem der vorangehenden Ansprüche, gekennzeichnet durch die Verwendung einer hohlen Sondenstange (1), die längs eines entfernbaren Führungsrohres (27) gleitet, wobei das Führungsrohr durch einen Dorn (26) oder ein Schnellbefestigungssystem in der Rückstossmasse (4) befestigt ist.

10. Vorrichtung nach einem der vorangehenden Ansprüche, dadurch gekennzeichnet, dass die Sondenstange aus mehreren Teilen durch ein Ineinandersteck-und-Verriegelungssystem nach Massgabe des Verlaufs der Bohrung zusammensetzbar ist.

11. Vorrichtung nach einem der vorangehenden Ansprüche, dadurch gekennzeichnet, dass das Gehäuse des Generators aus thermoplastischem oder aus Verbundmaterial besteht und sich ausserhalb seines Verwendungsgehäuses bei niedrigem Druck öffnet.

## Claims

1. Soil recognition and characterisation system comprising :
    - a recoil tube (7),
    - a probe rod (1) movable in comparison with the recoil tube (7),
    - a pyrotechnical generator,
    - detection means (6) consisting of several parts,
        . the probe rod (1) being destined to penetrate in the soil under the effect of the pyrotechnical generator (5),
        . the detection means (6) being fixed on the recoil tube (7) and each of their parts detecting the passing of the probe rod (1) at its level, so as to supply a movement measurement of the probe rod (1) during its penetration.

2. System according to claim 1, characterised in that the movement of the probe rod (1) is detected by the passing of a magnet (3) linked to this rod, through the solenoids (6) located along the outer tube (7).

3. System according to claim 1, characterised in that the movement of the probe rod (1) is detected by the passing of said rod (1) in front of photo-electric cells or electromechanical contactors.

4. System according to anyone of the preceding claims, characterised in that the rod movement coordinates are stored in a computer (9), specific or no, Pre-programmed and initialized with the system parameters, in order to acquire directly soil characteristics and to make them available for display or printing.

5. System according to anyone of the preceding claims, characterised in that the balance of the amount of movements transmitted to the probe rod is ensured by a recoiling mass (4) located in the recoil tube (7) and whose kinetic energy is absorbed by using the force of gravity.

6. System according to anyone of the preceding claims, characterised in that the recoiling mass serves as a guiding tube for the probe rod and as expansion chamber for gases.

7. System according to anyone of the preceding claims, characterised in that the recoiling mass comprises the housing of the pyrotechnical gas generator (5).

8. System according to anyone of the preceding claims, characterised in that the recoiling mass exhibits, at the level of the expansion chamber (8), one or several safety vents (28) and that the recoil tube exhibits a bore (29) to facilitate the gas exhaust in case the probe rod is prematurly blocked in the soil during penetration.

9. System according to anyone of the preceding claims, characterised by the use of a hollow probe rod (1) sliding along a removable guiding tube (27) which is blocked in the recoiling mass (4) through a chuck (26) or a quick fastener system.

10. System according to anyone of the preceding claims, characterised in that the probe rod can be made of several sections assembled by an encasing-locking-fitting as the probing progresses.

11. System according to anyone of the preceding claims, characterised in that the body of the generator is made of thermoplastic or composite materials and opens up at low pressure outside of its operating housing.

Figure 1

Figure 2

Figure 3

7

31

30

Figure 4